# FASCICULE DE BREVET EUROPEEN

(11) **EP 2 377 466 B1**
(45) Date de publication et mention de la délivrance du brevet: **08.07.2015**
(21) Numéro de dépôt: 11162024.1
(22) Date de dépôt: 12.04.2011
(51) Int. Cl.: A61B 6/00, A61B 17/225, A61B 6/08

(54) **Appareil de traitement par ondes de pression, équipé d'un système pour déplacer l'arceau d'un système d'imagerie par rayons X**
Gerät zur Behandlung durch Druckwellen, das mit einem System zum Verschieben des Bogens eines Bildgebungssystems durch Röntgenwellen ausgestattet ist
Pulse wave treatment device equipped with a system to move the archway of an X-ray imaging system

(30) Priorité: 13.04.2010 FR 1052791
(43) Date de publication de la demande: 19.10.2011
(73) Titulaire: EDAP TMS France, 69120 Vaulx-en-Velin (FR)
(72) Inventeur: Reboul, Pierre, 69570, DARDILLY (FR); Nallet, Olivier, 69003, LYON (FR); Peyrard, André, 42320, SAINT CHRISTO EN JAREZ (FR)
(74) Mandataire: Thibault, Jean-Marc

(56) Documents cités:
- EP-A1- 0 397 980
- EP-A1- 0 715 831
- WO-A1-02/00116
- DE-A1- 10 303 462

## Description

La présente invention concerne un appareil de destruction de concrétions intracorporelles solides pour des traitements de lithotritie.

De manière connue, un appareil de lithotritie comporte un générateur d'ondes de pression telles que des ondes de choc acoustiques permettant de détruire des concrétions ou des lithiases rénales, biliaires ou salivaires. L'appareil de lithotritie est associé à un dispositif d'imagerie permettant de détecter et de visualiser la présence de lithiases dans le corps d'un patient ou d'un animal. Les lithiases et le foyer du générateur sont mis en coïncidence à l'aide de systèmes de déplacement mécaniques permettant d'amener soit les lithiases sur le point de focalisation du générateur, soit le point de focalisation du générateur sur les lithiases.

Pour réaliser cette opération de mise en correspondance, il convient d'utiliser un système d'imagerie permettant de visualiser la lithiase et de déterminer son positionnement dans les trois dimensions de l'espace. Les systèmes d'imagerie par ultrasons et par rayons X sont les dispositifs d'imagerie les plus couramment utilisés.

Un dispositif d'imagerie par ultrasons permet de réaliser un plan image de deux dimensions sous la forme d'une coupe anatomique dont l'orientation est déterminée par l'opérateur manipulant une sonde échographique. Lorsque ce plan de coupe permet de visualiser la lithiase, il est possible de déterminer la position de celle-ci dans l'espace sous réserve de connaître la position et l'orientation de la sonde échographique ainsi que la position de la lithiase dans le plan image échographique.

La localisation spatiale d'une lithiase est sensiblement plus complexe à mener à bien à partir d'un système d'imagerie par rayons X du type arceau tel que celui décrit par les documents EP 0 715 831, DE 103 03 4b2 ou EP 0 397 980. Un système d'imagerie par rayons X comporte un arceau équipé à l'une de ses extrémités d'une source à rayons X et à l'autre de ses extrémités et en vis-à-vis, d'un capteur d'imagerie. Cet arceau est monté mobile en rotation autour d'un axe d'oscillation coupant perpendiculairement l'axe passant par la source de rayons X et le capteur d'imagerie. Un tel système d'imagerie permet de disposer d'une image en deux dimensions résultant d'une projection anatomique des structures biologiques sur le plan du capteur d'imagerie. Au contraire du système d'imagerie par ultrasons, cette image en deux dimensions ne contient pas les informations spatiales nécessaires à la localisation dans l'espace de la lithiase même en connaissant précisément la position de l'arceau. Pour disposer de cette information spatiale, il est nécessaire de réaliser une seconde image avec une incidence de l'arceau par rapport à la lithiase, différente, par exemple de quelques dizaines de degrés, par rapport à l'incidence de l'arceau prise lors de la première image. Les deux images prises représentent la lithiase selon deux projections différentes. En identifiant la lithiase sur chacune de ces deux images, il est possible par des méthodes de calcul connues ou par des méthodes d'alignement successives, de déterminer la position dans l'espace de la lithiase.

Bien entendu, la réalisation de ces deux clichés doit être effectuée consécutivement de manière que la position spatiale de la lithiase ne soit pas modifiée entre les deux clichés c'est-à-dire que le patient soit resté immobile. En pratique, l'opérateur doit positionner l'arceau dans une première position, réaliser un premier cliché puis changer l'incidence de l'arceau pour le placer dans une seconde position d'incidence afin de réaliser le second cliché.

Il est à noter que l'opérateur ne peut pas rester à proximité de la source à rayons X lorsque celle-ci émet des rayons pour la prise des clichés. Aussi, pour chacun des clichés, l'opérateur doit se protéger derrière un écran de protection puis revenir vers l'équipement afin de modifier l'incidence de l'arceau et à nouveau s'en éloigner pour se protéger de l'émission résultant de ce cliché. En pratique, la prise des deux clichés est relativement longue à mener à bien, de sorte que fréquemment le patient a bougé entre les deux clichés, de sorte qu'il apparaît nécessaire de recommencer la procédure de prise d'images.

Pour tenter de résoudre ce problème, il a été proposé, dans l'état de la technique, un système d'imagerie par rayons X dont l'arceau est motorisé pour éviter l'intervention d'un opérateur pour la modification d'incidence de l'arceau entre la prise des deux images. Un tel système d'imagerie par rayons X s'avère très coûteux et n'apparaît pas, en pratique, adapté pour être associé à un appareil de lithotritie. En effet, la commande de la motorisation de l'arceau est généralement située sur le bâti du système d'imagerie. Ainsi, il apparaît une difficulté à l'opérateur pour commander à la fois le système d'imagerie et l'appareil de lithotritie.

La présente invention vise donc à remédier aux inconvénients de l'état de la technique en proposant une solution permettant de pouvoir placer l'arceau d'un système d'imagerie par rayons X dans deux positions stables, de manière automatique, simple et peu onéreuse, lorsqu'un tel système d'imagerie est utilisé en association avec un appareil de lithotritie.

Un autre objet de l'invention est de proposer une solution de motorisation de l'arceau d'un système d'imagerie par rayons X, permettant d'utiliser ce système d'imagerie indépendamment de l'appareil de lithotritie.

Pour atteindre de tels objectifs, l'objet de l'invention concerne un appareil de traitement par ondes de pression comportant un châssis supportant un générateur d'ondes de pression.

L'invention est définie dans les revendications annexées.

Selon l'invention, l'appareil de traitement comporte :
- un système d'actionnement comportant une partie fixe montée sur le châssis et une partie mobile en translation selon une direction sensiblement perpendiculaire à un axe d'oscillation d'un arceau faisant partie d'un système d'imagerie qui est indépendant par rapport à l'appareil de traitement,
- la partie mobile en translation exerçant un effort de poussée ou de traction, décalé par rapport à l'axe d'oscillation,
- un système de liaison avec l'arceau, comportant une structure d'assemblage démontable entre l'arceau et la partie mobile en translation du système d'actionnement,
- et une unité de commande du système d'actionnement pour placer le système de liaison dans au moins deux positions stables correspondant à deux positions différentes d'incidence de l'arceau du système d'imagerie.

De plus, l'appareil de traitement selon l'invention peut présenter en outre en combinaison au moins l'une et/ou l'autre des caractéristiques additionnelles suivantes :
- un dispositif de contrôle de la course de la partie mobile du système d'actionnement, ce dispositif de contrôle étant relié à l'unité de commande,
- la structure d'assemblage démontable comporte un étrier muni de vis de pression,
- le système de liaison avec l'arceau est relié à la partie mobile du système d'actionnement par l'intermédiaire d'une structure articulée,
- la structure articulée comporte un axe d'articulation sensiblement horizontal et sensiblement parallèle à l'axe d'oscillation de l'arceau et un axe d'articulation sensiblement vertical autorisant une rotation verticale et/ou horizontale de la partie mobile du système d'actionnement par rapport à la structure d'assemblage,
- la partie fixe du système d'actionnement est montée sur le châssis par intermédiaire d'une structure d'articulation,
- la structure d'articulation comporte un axe d'articulation sensiblement horizontal et un axe d'articulation sensiblement vertical autorisant une rotation verticale et/ou horizontale de la partie fixe du système d'actionnement par rapport au châssis,
- le système d'actionnement est monté sur le châssis de manière que la direction de translation de la partie mobile se trouve écartée de l'axe d'oscillation de l'arceau,
- le système d'actionnement est monté sur le châssis pour s'étendre en dessous du générateur d'ondes de pression,
- le châssis est pourvu de parois de protection dont au moins une comporte une ouverture de passage pour l'organe mobile du système d'actionnement.

L'appareil de traitement selon l'invention associé à un système d'imagerie du type à arceau forment ensemble un équipement médical permettant le traitement des lithiases par ondes de pression,

L'équipement comporte ainsi :
- un système d'imagerie comportant un arceau monté oscillant selon un axe d'oscillation,
- et un appareil de traitement par ondes de pression indépendant par rapport au système d'imagerie et comportant notamment un système de liaison avec l'arceau comportant une structure d'assemblage démontable entre l'arceau et une partie mobile en translation d'un système d'actionnement faisant partie de l'appareil de traitement.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective montrant un appareil de traitement par ondes de pression conforme à l'invention.
Les **Figures 2A** et **2B** sont des vues illustrant l'appareil de traitement dans deux positions caractéristiques de positionnement de l'arceau d'un système d'imagerie.
Les **Figures 3** et **4** sont des vues de détail respectivement de face et de côté de la liaison entre l'arceau du système d'imagerie et l'appareil de traitement.
La **Figure 5** est une vue de détail montrant la fixation du système d'actionnement sur le châssis de l'appareil de traitement.

Tel que cela ressort plus précisément des Figures, l'objet de l'invention concerne un appareil de traitement **1** par ondes de pression, de concrétions ou de lithiases intracorporelles localisées dans le corps d'un patient ou d'un animal à l'aide d'un système d'imagerie **2** du type par rayons X.

De manière classique, un système d'imagerie **2** par rayons X comporte un arceau **3** demi-circulaire équipé à une extrémité, d'une source **4** de rayons X et à son autre extrémité d'un capteur d'imagerie **5.** Dans l'exemple illustré, l'arceau **3** présente une face principale **3₁** plane de profil incurvé et bordée de part et d'autre par des faces latérales **3₂.** La source **4** de rayons X et le capteur d'imagerie **5** sont situés en vis-à-vis l'un de l'autre selon une direction générale **X** en étant écartés pour permettre le positionnement d'un patient. L'arceau **3** est monté mobile en rotation par rapport à un châssis **6** selon un axe d'oscillation **7** s'étendant selon une direction horizontale **Y.** L'axe d'oscillation **7** est situé au milieu de l'arceau **3** de sorte que la direction horizontale **Y** de l'axe d'oscillation **7** coupe perpendiculairement la direction commune **X** d'extension de la source **4** de rayons X et du capteur d'imagerie **5.**

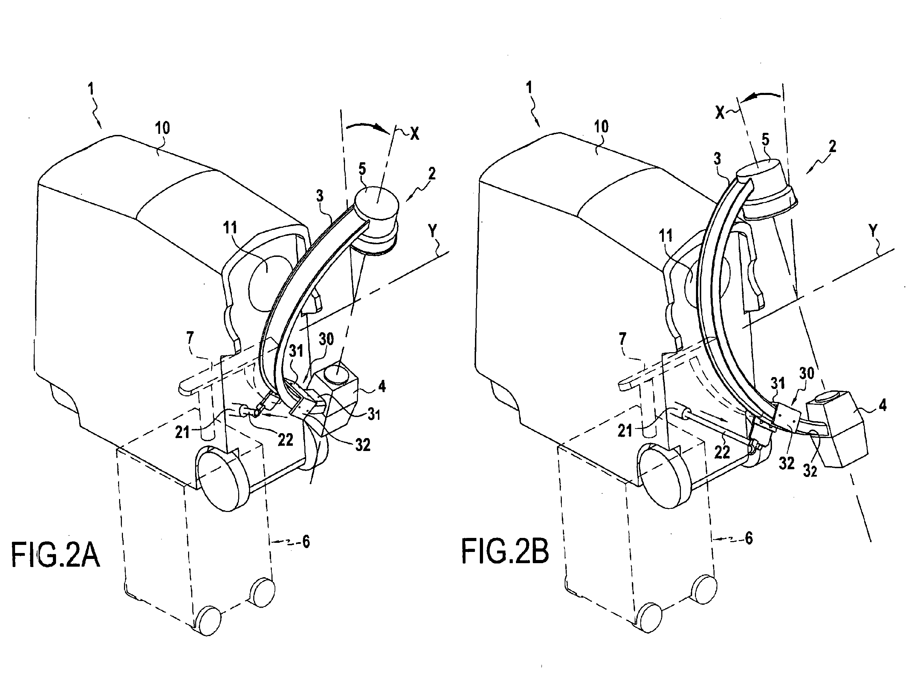

L'arceau **3** peut ainsi occuper, comme cela ressort plus précisément des **Fig. 2A** et **2B****,** deux positions stables d'incidence différentes dans chacune desquelles un cliché peut être pris d'une personne disposée entre la source **4** de rayons X et le capteur d'imagerie **5.** Par exemple, le décalage d'incidence entre les deux positions illustrées aux **Fig. 2A** et **2B** qui est obtenu par la rotation de l'arceau **3** autour de l'axe d'oscillation **7,** est de l'ordre de quelques dizaines de degrés. Le système d'imagerie **2** n'est pas décrit plus précisément car il est bien connu de l'homme du métier et ne fait pas partie précisément de l'objet de l'invention.

L'appareil de traitement **1** selon l'invention tel qu'un appareil de lithotritie comporte un bâti ou un châssis **10** équipé d'un générateur **11** d'ondes de pression telles que des ondes de choc acoustiques. Dans l'exemple de réalisation illustré, le châssis **10** présente une forme sensiblement parallélépipédique et se trouve avantageusement enveloppée par un capotage ou des parois de protection. Par exemple, le châssis **10** est ainsi équipé de deux parois de côté **10₁** s'étendant sensiblement parallèlement entre elles et reliées entre elles d'un côté par une paroi arrière **10₂** et de l'autre côté par une paroi avant **10₃.** Les parois de côté **10₁** et les parois avant **10₃** et arrière **10₂** sont réunies entre elles à leur partie supérieure par une paroi de dessus **10₄.** De manière classique, le châssis **10** est équipé de roues **13** permettant le roulement et le déplacement du châssis **10.**

De façon classique, le générateur **11** est adapté pour émettre des ondes de choc acoustiques dirigées vers un foyer cible que l'on cherche à mettre en coïncidence par l'intermédiaire du système d'imagerie **2,** avec les concrétions en vue de détruire ces dernières. De manière conventionnelle, le générateur **11** comporte un réflecteur ellipsoïdal dans lequel est positionnée une électrode qui permet de générer une onde de choc acoustique au premier foyer du réflecteur ellipsoïdal. Par la géométrie particulière du réflecteur, l'onde de choc créée au premier foyer se trouve réfléchie au second foyer du réflecteur, le second foyer se trouvant à l'extérieur du générateur, ce qui permet de viser des concrétions situées dans le corps d'un patient ou d'un animal. Le générateur **11** n'est pas décrit plus précisément car il ne fait pas partie précisément de l'invention et sa réalisation est bien connue de l'homme du métier.

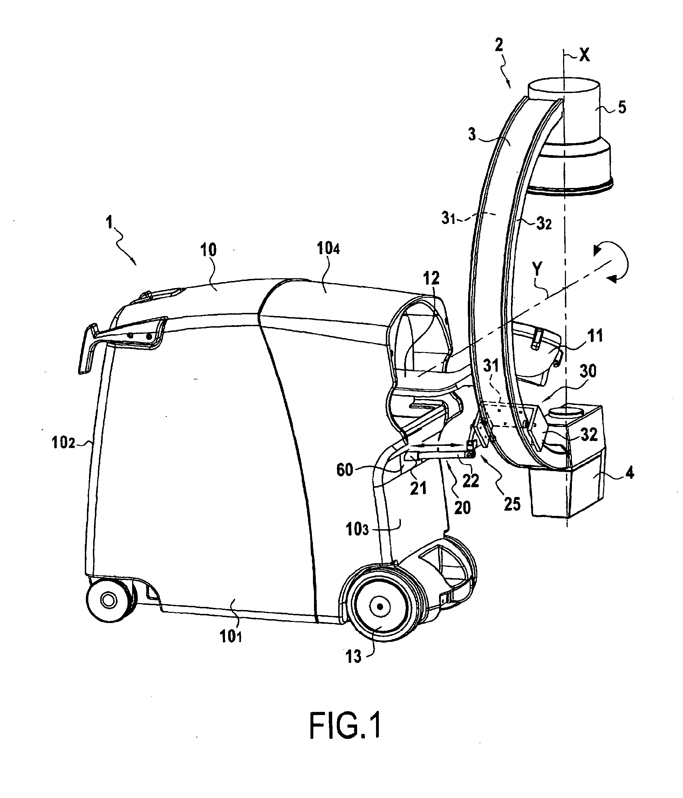

Dans l'exemple illustré, le générateur **11** est monté sur une structure de guidage **12** permettant de le déplacer entre une position rentrée à l'intérieur du châssis **10 (****Fig. 2A**, **2B**) et une position de sortie ou de traitement **(****Fig. 1****)** dans laquelle le générateur **11** est situé à proximité du patient. Bien entendu, le générateur **11** peut être monté de manière différente sur le châssis **10** en étant déplacé ou non par rapport au châssis **10.**

Conformément à l'invention, l'appareil de traitement **1** comporte un système d'actionnement **20** pour l'arceau **3** du système d'imagerie **2 (****Fig.1****).** Le système d'actionnement **20** agit sur l'arceau **3** afin d'assurer sa rotation autour de l'axe d'oscillation **7** de manière à obtenir le positionnement de l'arceau **3** dans deux positions stables présentant des incidences différentes **(****Fig. 2A**, **2B**). Le système d'actionnement **20** comporte une partie fixe **21** monté sur le châssis **10** et une partie mobile en translation **22** selon une direction sensiblement perpendiculaire à l'axe d'oscillation **7.** Le système d'actionnement **20** exerce ainsi un effort de poussée ou de traction sensiblement perpendiculaire à l'axe d'oscillation **7.** Le point d'application de cet effort de poussée ou de traction sur l'arceau **3** est bien entendu, décalé par rapport à l'axe d'oscillation **7** afin d'obtenir la rotation de l'arceau **3** autour de l'axe d'oscillation **7.**

Selon une variante préférée de réalisation, le système d'actionnement **20** comporte un vérin dont le corps **21** est fixé sur le châssis tandis que la tige **22** est déplacée en translation pour permettre l'oscillation de l'arceau **3.** Le vérin **20** peut être de toute nature, par exemple électrique, pneumatique ou hydraulique. Bien entendu, le système d'actionnement **20** peut être réalisé de manière différente, comme par exemple par un actionneur à mouvement linéaire intégrant ou non un système de transformation de mouvement.

L'appareil de traitement **1** comme illustré à la **Fig. 1****,** comporte également un système **25** de liaison avec l'arceau **3,** relié à la partie mobile **22** du système d'actionnement **20.** Ce système **25** assure ainsi une liaison ou un assemblage démontable ou temporaire entre le système d'actionnement **20** et l'arceau **3** pour permettre de placer ce dernier dans au moins deux positions stables correspondant à deux positions différentes d'incidence de l'arceau **3.** L'appareil de traitement **1** et le système d'imagerie **2** sont conçus pour être indépendants l'un de l'autre ou être complètement séparés de manière que le système d'imagerie **2** puisse être utilisé alors que l'appareil de traitement **1** est séparé du système d'imagerie. Lorsque le besoin s'en fait sentir, le système de liaison **25** assure un assemblage temporaire démontable entre l'arceau **3** du système d'imagerie **2** et l'appareil de traitement **1.**

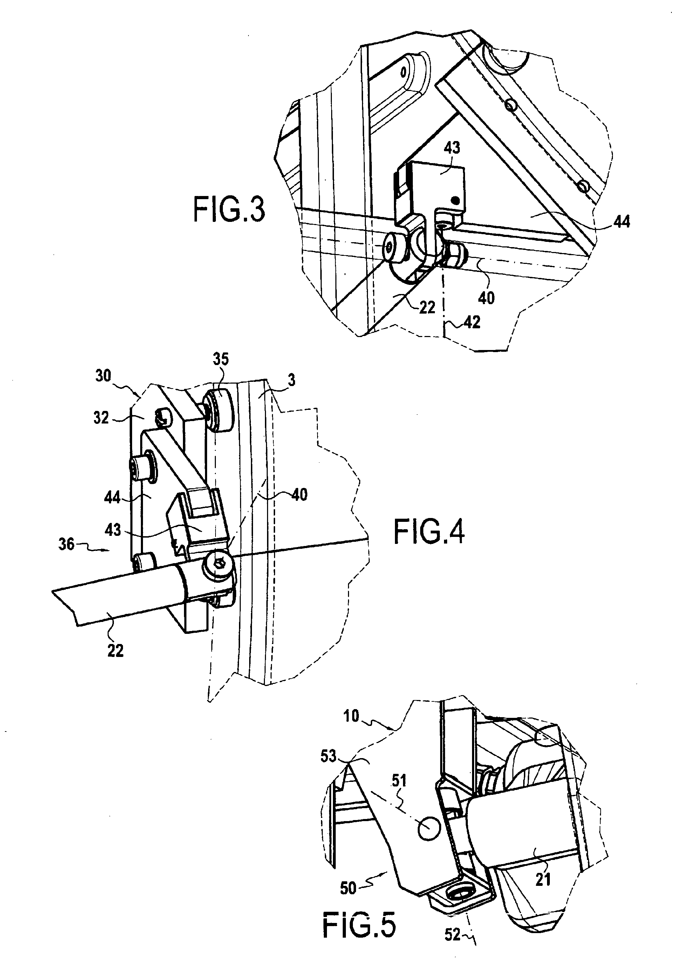

Selon une variante de réalisation, le système de liaison **25** comporte une structure d'assemblage démontable **30** entre l'arceau **3** et la partie mobile **22** du système d'actionnement **20. (****Fig. 2A, Fig. 2B****).** Dans l'exemple de réalisation illustré, la structure d'assemblage démontable **30** se présente sous la forme d'un étrier comportant une âme centrale **31** prolongée de part et d'autre et à l'équerre, par deux ailes **32.** L'étrier **30** est monté pour venir chevaucher ou s'emboîter sur l'arceau **3** de manière que les ailes **32** s'étendent de part et d'autre des faces latérales **3₂** de l'arceau tandis que l'âme **31** recouvre la face principale **3₁.** L'étrier **30** est fixé sur l'arceau **3** à l'aide de vis de pression **35** portées par les ailes **32 (****Fig. 4****).** L'étrier **30** peut facilement être solidarisé ou désolidarisé par rapport à l'arceau **3** par respectivement le serrage et le desserrage des vis de pression **35.** En d'autres termes, le système d'imagerie **2** peut facilement être rendu indépendant par rapport à l'appareil de traitement **1** dans la mesure où le système de liaison **25** est relié à la partie mobile **22** à l'aide d'une structure d'assemblage à caractère démontable. Il est à noter que la conception de la structure d'assemblage **30** (étrier et vis de pression) permet de s'adapter, sans modification, à tous types d'arceaux **3.**

Selon une variante préférée de réalisation, le système de liaison **25** comporte entre l'arceau **3** et la partie mobile **22** du système d'actionnement **20** une structure articulée **36** adaptée pour assurer la rotation de l'arceau **3** lorsque ce dernier est soumis à un effort de déplacement linéaire perpendiculaire à l'axe d'oscillation **7.** Tel que cela ressort plus précisément de l'exemple illustré aux **Fig. 3** et **4****,** la structure articulée **36** est interposée entre la partie mobile **22** du système d'actionnement **20** et l'étrier **30.** Cette structure articulée **36** comporte un axe d'articulation **40** sensiblement horizontal et sensiblement parallèle à l'axe d'oscillation **7** de l'arceau et un axe d'articulation **42** sensiblement vertical autorisant en combinaison, une rotation verticale et/ou horizontale de la partie mobile **22** du système d'actionnement **20** par rapport à l'étrier **30.** Dans l'exemple illustré, l'axe d'articulation horizontal **40** est monté entre l'extrémité de la partie mobile **22** et une pièce de liaison intermédiaire **43** apte à pivoter autour de cet axe d'articulation horizontal **40.** L'axe d'articulation vertical **42** est monté entre la pièce de liaison intermédiaire **43** et un flasque **44** fixé à une aile **32** de l'étrier **30.**

Avantageusement, la pièce de liaison intermédiaire **43** est montée à l'aide d'un moyen de montage démontable (de préférence sans outil) par rapport au flasque **44** facilitant ainsi l'opération de mise en place et d'enlèvement de l'étrier **30.** De plus, le système de liaison **25** peut être désolidarisé du système d'actionnement **20** favorisant ainsi le repliage de la tige **22** du vérin.

De préférence, le vérin **20** est monté de manière qu'en position rentrée de la tige, le vérin **20** se trouve complètement escamoté à l'intérieur du châssis **10.**

Selon une variante préférée de réalisation, la partie fixe **21** du système d'actionnement **20** est montée sur le châssis **10** par l'intermédiaire d'une structure d'articulation **50.** Par exemple, la structure d'articulation **50** comporte un axe d'articulation sensiblement horizontal **51** et un axe d'articulation sensiblement vertical **52** autorisant ensemble une rotation verticale et/ou horizontale de la partie fixe **21** du système d'actionnement **20** par rapport au châssis **10.** Dans l'exemple illustré à la **Fig. 5****,** l'axe d'articulation horizontal **51** est monté entre le corps **21** du vérin et les deux branches d'un étrier **53** présenté par le châssis **10.** L'axe d'articulation vertical **52** est monté entre le corps **21** du vérin et l'étrier **53.** Ces deux degrés de liberté de rotation du vérin **20** par rapport au châssis **10** sont mis en oeuvre lors de l'opération de déplacement de l'arceau **3.**

L'appareil de traitement **1** comporte également une unité de commande non représenté, du système d'actionnement **20** permettant de placer le système de liaison **25** dans au moins deux positions stables correspondant à deux positions différentes d'incidence de l'arceau **3.** Par exemple, cette unité de commande pilote le système d'actionnement **20** par l'envoi de signaux électriques de commande. Avantageusement, cette unité de commande fait partie de l'électronique de commande et de gestion de l'appareil de traitement **1.** Ainsi, les ordres de déplacement et d'arrêt du système d'actionnement **20** peuvent être pilotés à partir de l'électronique de commande et de gestion de l'appareil de traitement **1.**

Selon une variante préférée de réalisation, cette unité de commande est reliée à un dispositif de contrôle de la course de la partie mobile **22** du système d'actionnement **20.** Un tel dispositif de contrôle est destiné à informer l'opérateur de la course de la partie mobile **22** ou des risques potentiels de collision avec un équipement situé dans l'environnement et/ou à interrompre le déplacement du système d'actionnement **20.** Le dispositif de contrôle comporte notamment des capteurs de déplacement, des capteurs d'effort et/ou des capteurs de proximité.

Selon une variante préférée de réalisation, le système d'actionnement **20** est monté sur le châssis **10** de manière que la direction de translation de la partie mobile **22** se trouve écartée de l'axe d'oscillation **7** de l'arceau **3** pour minimiser les efforts à appliquer sur l'arceau **3.** En d'autres termes, le système d'actionnement **20** est monté de manière que le point d'application de l'effort de poussée ou de traction exercé par le système d'actionnement **20** soit le plus éloigné possible de l'axe d'oscillation **7** pour minimiser les efforts à exercer en vue d'obtenir l'oscillation souhaitée pour l'arceau **3.** Le point d'application peut être situé au-dessus ou au-dessous de l'axe d'oscillation **7** situé au milieu de l'arceau **3.**

Dans l'exemple préféré illustré sur les dessins, le système d'actionnement **20** est monté sur le châssis **10** de manière que le point d'application de l'effort se trouve situé en dessous de l'axe d'oscillation **7.** Selon cette variante préférée mais non exclusive, le système d'actionnement **20** est monté sur le châssis **10** pour s'étendre en dessous du générateur **11.** Ainsi, le générateur **11** et la partie mobile **22** sont situés l'un au-dessus de l'autre, au niveau de la paroi avant **10₃.** Cette paroi avant **10₃** comporte ainsi une ouverture de passage **60** pour l'organe mobile **22** du système d'actionnement **20.**

Il ressort de la description qui précède que l'appareil de traitement **1** permet d'assurer ses fonctions classiques de traitement de lithiases tout en permettant de commander la rotation de l'arceau **3** d'un système d'imagerie **2** pour le placer automatiquement dans deux positions caractéristiques de prises d'images. L'appareil de traitement **1** et le système de prise d'images **2** forment ensemble un équipement adapté pour le traitement de lithiases par ondes de pression. Compte tenu de l'assemblage amovible entre l'appareil de traitement **1** et le système d'imagerie **2,** ce dernier peut être utilisé indépendamment d'un appareil de lithotritie. Par ailleurs, compte tenu du montage amovible entre le système de liaison **25** et le système d'actionnement **20,** ce dernier peut être monté pour être complètement rentré à l'intérieur du châssis **10** lorsque la commande en rotation de l'arceau d'un système d'imagerie **2** n'est pas requise.

L'invention n'est pas limitée aux exemples décrits et représentés car diverses modifications peuvent y être apportées sans sortir de son cadre.

## Revendications

1. Appareil de traitement par ondes de pression comportant un châssis (**10**) supportant un générateur d'ondes de pression (**11**), comportant:
- un système d'actionnement (**20**) comportant une partie fixe (**21**) montée sur le châssis (**10**) et une partie mobile en translation (**22**) selon une direction sensiblement perpendiculaire à un axe d'oscillation (**7**) d'un arceau (**3**) faisant partie d'un système d'imagerie (**2**) qui est indépendant par rapport à l'appareil de traitement,
- la partie mobile en translation (22) exerçant un effort de poussée ou de traction sur l'arceau, décalé par rapport à l'axe d'oscillation (7),
- un système (**25**) de liaison avec l'arceau (**3**), comportant une structure d'assemblage démontable (**35**) entre l'arceau (**3**) et la partie mobile en translation (**22**) du système d'actionnement (**20**),
- et une unité de commande du système d'actionnement (**20**) pour placer le système de liaison (**25**) dans au moins deux positions stables correspondant à deux positions différentes d'incidence de l'arceau (**3**) du système d'imagerie.

2. Appareil de traitement selon la revendication 1, **caractérisé en ce qu'**il comporte un dispositif de contrôle de la course de la partie mobile (**22**) du système d'actionnement (**20**), ce dispositif de contrôle étant relié à l'unité de commande.

3. Appareil de traitement selon la revendication 1 ou 2, **caractérisé en ce que** la structure d'assemblage démontable comporte un étrier (**30**) muni de vis de pression (**35**).

4. Appareil de traitement selon la revendication 1, **caractérisé en ce que** le système de liaison (**25**) avec l'arceau (**3**) est relié à la partie mobile (**22**) du système d'actionnement (**20**) par l'intermédiaire d'une structure articulée (**36**).

5. Appareil de traitement selon la revendication 4, **caractérisé en ce que** la structure articulée (**36**) comporte un axe d'articulation (**40**) sensiblement horizontal et sensiblement parallèle à l'axe d'oscillation (**7**) de l'arceau et un axe d'articulation (**42**) sensiblement vertical autorisant une rotation verticale et/ou horizontale de la partie mobile (**22**) du système d'actionnement (**20**) par rapport à la structure d'assemblage (**30**, **35**).

6. Appareil de traitement selon la revendication 1, **caractérisé en ce que** la partie fixe (**21**) du système d'actionnement (**20**) est montée sur le châssis par l'intermédiaire d'une structure d'articulation (**50**).

7. Appareil de traitement selon la revendication 6, **caractérisé en ce que** la structure d'articulation (**50**) comporte un axe d'articulation sensiblement horizontal (**51**) et un axe d'articulation sensiblement vertical (**52**) autorisant une rotation verticale et/ou horizontale de la partie fixe (**21**) du système d'actionnement par rapport au châssis (**10**).

8. Appareil de traitement selon la revendication 1, **caractérisé en ce que** le système d'actionnement (**20**) est monté sur le châssis (**10**) de manière que la direction de translation de la partie mobile (**22**) se trouve écartée de l'axe d'oscillation (**7**) de l'arceau.

9. Appareil de traitement selon la revendication 1, **caractérisé en ce que** le système d'actionnement (**20**) est monté sur le châssis pour s'étendre en dessous du générateur d'ondes de pression (**11**).

10. Appareil de traitement selon l'une des revendications 1 à 9, **caractérisé en ce que** le châssis (**10**) est pourvu de parois de protection dont au moins une (**10₃**) comporte une ouverture de passage (**60**) pour la partie mobile (**22**) du système d'actionnement (**20**).

11. Appareil de traitement selon l'une des revendications 1 à 10, **caractérisé en ce que** le système d'actionnement (**20**) comporte un vérin.

12. Equipement pour réaliser un traitement par ondes de pression, **caractérisé en ce qu'**il comporte :
- un système d'imagerie (**2**) comportant un arceau (**3**) monté oscillant selon un axe d'oscillation (7),
- et un appareil de traitement (**1**) par ondes de pression conforme à l'une des revendications 1 à 11, indépendant par rapport au système d'imagerie (**2**) et comportant notamment un système (**25**) de liaison avec l'arceau (**3**) comportant une structure d'assemblage démontable (**35**) entre l'arceau (**3**) et une partie mobile en translation (**22**) d'un système d'actionnement (**20**) faisant partie de l'appareil de traitement (**1**).

## Patentansprüche

1. Gerät zur Behandlung mit Druckwellen, umfassend ein Gestell (10), das einen Druckwellengenerator (11) trägt, umfassend:
- ein Betätigungssystem (20), umfassend einen festen Teil (21), der auf dem Gestell (10) montiert ist, und einen Teil (22), der in Translation in eine Richtung im Wesentlichen senkrecht auf die Schwingungsachse (7) eines Bogens (3), der Teil eines Bildgebungssystems (2) ist, das von dem Behandlungsgerät unabhängig ist, beweglich ist, wobei der in Translation bewegliche Teil (22) eine Schub- oder Zugkraft auf den Bogen ausübt, die in Bezug zur Schwingungsachse (7) versetzt ist,
- ein Verbindungssystem (25) mit dem Bogen (3), umfassend eine demontierbare Verbindungsstruktur (35) zwischen dem Bogen (3) und dem in Translation beweglichen Teil (22) des Betätigungssystems (20),
- und eine Steuereinheit des Betätigungssystems (20), um das Verbindungssystem (25) in mindestens zwei stabilen Positionen entsprechend zwei unterschiedlichen Eintreffpositionen des Bogens (3) des Bildgebungssystems anzuordnen.

2. Behandlungsgerät nach Anspruch 1, **dadurch gekennzeichnet, dass** es eine Vorrichtung zur Kontrolle des Hubs des beweglichen Teils (22) des Betätigungssystems (20) umfasst, wobei diese Kontrollvorrichtung mit der Steuereinheit verbunden ist.

3. Behandlungsgerät nach Anspruch 1 oder 2, **dadurch gekennzeichnet, dass** die demontierbare Verbindungsstruktur einen Bügel (30) umfasst, der mit Druckschrauben (35) versehen ist.

4. Behandlungsgerät nach Anspruch 1, **dadurch gekennzeichnet, dass** das Verbindungssystem (25) mit dem Bogen (3) mit dem beweglichen Teil (22) des Betätigungssystems (20) mit Hilfe einer Gelenkstruktur (36) verbunden ist.

5. Behandlungsgerät nach Anspruch 4, **dadurch gekennzeichnet, dass** die Gelenkstruktur (36) eine im Wesentlichen horizontale Gelenkachse (40) im Wesentlichen parallel zur Schwingungsachse (7) des Bogens und eine im Wesentlichen vertikale Gelenkachse (42) umfasst, die eine vertikale und/oder horizontale Drehung des beweglichen Teils (22) des Betätigungssystems (20) in Bezug zur Verbindungsstruktur (30, 35) gestattet.

6. Behandlungsgerät nach Anspruch 1, **dadurch gekennzeichnet, dass** der feste Teil (21) des Betätigungssystems (20) auf dem Gestell mit Hilfe einer Gelenkstruktur (50) montiert ist.

7. Behandlungsgerät nach Anspruch 6, **dadurch gekennzeichnet, dass** die Gelenkstruktur (50) eine im Wesentlichen horizontale Gelenkachse (51) und eine im Wesentlichen vertikale Gelenkachse (52) umfasst, die eine vertikale und/oder horizontale Drehung des festen Teils (21) des Betätigungssystems in Bezug zum Gestell (10) gestatten.

8. Behandlungsgerät nach Anspruch 1, **dadurch gekennzeichnet, dass** das Betätigungssystem (20) auf dem Gestell (10) derart montiert ist, dass die Translationsrichtung des beweglichen Teils (22) von der Schwingungsachse (7) des Bogens entfernt ist.

9. Behandlungsgerät nach Anspruch 1, **dadurch gekennzeichnet, dass** das Betätigungssystem (20) auf dem Gestell derart montiert ist, dass es sich unter dem Druckwellengenerator (11) erstreckt.

10. Behandlungsgerät nach einem der Ansprüche 1 bis 9, **dadurch gekennzeichnet, dass** das Gestell (10) mit Schutzwänden versehen ist, von denen mindestens eine (10₃) eine Durchgangsöffnung (60) für den beweglichen Teil (22) des Betätigungssystems (20) umfasst.

11. Behandlungsgerät nach einem der Ansprüche 1 bis 10, **dadurch gekennzeichnet, dass** das Betätigungssystem (20) einen Zylinder umfasst.

12. Ausrüstung zur Durchführung einer Behandlung mit Druckwellen, **dadurch gekennzeichnet, dass** sie umfasst:
- ein Bildgebungssystem (2), umfassend einen Bogen (3), der schwingend entlang einer Schwingungsachse (7) montiert ist,
- und ein Gerät (1) zur Behandlung mit Druckwellen nach einem der Ansprüche 1 bis 11, das von dem Bildgebungssystem (2) unabhängig ist und insbesondere ein Verbindungssystem (25) mit dem Bogen (3) besitzt, umfassend eine demontierbare Verbindungsstruktur (35) zwischen dem Bogen (3) und einem in Translation beweglichen Teil (22) eines Betätigungssystems (20), das Teil des Behandlungsgeräts (1) ist.

## Claims

1. Treatment equipment by pressure waves comprising a chassis (10) supporting a pressure-wave generator (11), comprising:
- an actuation system (20) comprising a fixed part (21) mounted on the chassis (10) and a mobile part in translation (22) according to a direction substantially perpendicular to an axis of oscillation (7) of a curved section (3) being part of an imaging system (2) which is independent relative to the treatment equipment, the mobile part in translation (22) exerting a thrust or a traction force on the curved section, offset relative to the axis of oscillation (7).
- a linking system (25) with the curved section (3), comprising a disassemblable assembly structure (35) between the curved section (3) and the mobile part in translation (22) of the actuation system (20),
- and a control unit of the actuation system (20) for placing the linking system (25) in at least two stable positions corresponding to two different positions of incidence of the curved section (3) of the imaging system.

2. The treatment equipment as claimed in Claim 1, **characterised in that** it comprises a control device of the course of the mobile part (22) of the actuation system (20), this control device being connected to the control unit.

3. The treatment equipment as claimed in Claim 1 or 2, **characterised in that** the disassemblable assembly structure comprises a clamp (30) fitted with a pressure screw (35).

4. The treatment equipment as claimed in Claim 1, **characterised in that** the linking system (25) with the curved section (3) is connected to the mobile part (22) of the actuation system (20) by means of an articulated structure (36).

5. The treatment equipment as claimed in Claim 4, **characterised in that** the articulated structure (36) comprises an axis of articulation (40) substantially horizontal and substantially parallel to the axis of oscillation (7) of the curved section and a substantially vertical axis of articulation (42) allowing vertical and/or horizontal rotation of the mobile part (22) of the actuation system (20) relative to the assembly structure (30, 35).

6. The treatment equipment as claimed in Claim 1, **characterised in that** the fixed part (21) of the actuation system (20) is mounted on the chassis by means of an articulation structure (50).

7. The treatment equipment as claimed in Claim 6, **characterised in that** the articulation structure (50) comprises a substantially horizontal axis of articulation (51) and a substantially vertical axis of articulation (52) allowing vertical and/or horizontal rotation of the fixed part (21) of the actuation system relative to the chassis (10).

8. The treatment equipment as claimed in Claim 1, **characterised in that** the actuation system (20) is mounted on the chassis (10) such that the direction of translation of the mobile part (22) is away from the axis of oscillation (7) of the curved section.

9. The treatment equipment as claimed in Claim 1, **characterised in that** the actuation system (20) is mounted on the chassis to extend below the pressure-wave generator (11).

10. The treatment equipment as claimed in any one of Claims 1 to 9, **characterised in that** the chassis (10) is provided with protective walls whereof at least one (10₃) comprises a passage opening (60) for the mobile part (22) of the actuation system (20).

11. The treatment equipment as claimed in any one of Claims 1 to 10, **characterised in that** the actuation system (20) comprises a jack.

12. Equipment for executing treatment by pressure waves, **characterised in that** it comprises:
- an imaging system (2) comprising a curved section (3) mounted oscillating according to an axis of oscillation (7),
- and treatment equipment (1) by pressure waves as claimed in any one of Claims 1 to 11, independent relative to the imaging system (2) and comprising especially a linking system (25) with the curved section (3) comprising a disassemblable assembly structure (35) between the curved section (3) and a mobile part in translation (22) of an actuation system (20) forming part of the treatment equipment (1).
